# EUROPEAN PATENT APPLICATION

(11) **EP 2 550 969 A1**
(43) Date of publication of application: **30.01.2013**
(21) Application number: 12178299.9
(22) Date of filing: 27.07.2012
(51) Int. Cl.: A61K 36/66, G01N 33/50

(54) **METHOD OF DIAGNOSING ALLERGY OR HYPERSENSITIVITY TO OPIATES AND TO STRUCTURALLY RELATED COMPOUNDS.**

(30) Priority: 29.07.2011 ES 201131321; 07.06.2012 ES 201230889
(71) Applicant: Diater Laboratorios, 28918 Leganés - Madrid (ES)
(72) Inventor: Palacios Peláez, Ricardo, E-28918 Leganés - Madrid (ES); Aldana Sánchez, Dulce, E-28918 Leganés - Madrid (ES); Pineda De La Losa, Fernando, E-28918 Leganés - Madrid (ES); Armentia Medina, Alicia, E-47014 Valladolid (ES); Martín Armentia, Blanca, E-47014 Valladolid (ES); De Lecea Flores De Lemus, Carlos, E-08023 Barcelona (ES); Puente Crespo, Yolanda, E-41011 Sevilla (ES)
(74) Representative: ABG Patentes, S.L.

(57) **Abstract**

The present invention relates to water soluble and fat soluble *Papaver somniferum* seed extracts, to their production methods and to their use for diagnosing allergy or hypersensitivity to an opiate or to a structurally related compound.

## Description

### Field of the Invention

The invention is generally encompassed within the area of allergy diagnosis; particularly in a method for diagnosing allergy or hypersensitivity to opiates and structurally related compounds, particularly opiates used in anesthesia, and neuromuscular blocking agents (NMBAs) based on the use of water soluble and/or fat soluble *Papaver somniferum* seed extracts.

### Background of the Invention

Anesthesia is a controlled medical act in which drugs are used for blocking a patient's pain and touch sensitivity, either in his/her entire body or part of it and with or without compromising consciousness. General anesthesia is characterized by causing hypnosis, amnesia, analgesia, muscle relaxation and loss of reflexes. To that end, anesthesiologists administer various drugs to patients. Hypnotics, strong analgesics, muscle relaxants and other substances, for example, anticholinergics, benzodiazepines and acetylcholinesterases reversing the effect of muscle relaxants are often administered in general anesthesia.

Said analgesics include natural opiates (morphine) or synthetic opiates (fentanyl, meperidine, alfentanil and remifentanil), and morphine-related compounds, for example, codeine, heroin, methadone and pholcodine. Muscle relaxants include the so-called NMBAs (neuromuscular blocking agents) which have a quaternary ammonium ion structure and contain a hydrophobic ring skeleton and a hydrophilic tertiary amine [Fischer MM. et al., Immunoassays for the diagnosis of anaphylaxis to neuromuscular blocking drugs: the value of morphine for the detection of IgE antibodies in allergic subjects. Anaesth Intensive Care 2000; 28:167-70], a structure similar to that of morphine.

The most common cause (50 - 70%) of anaphylactic reactions during anesthesia mainly involves said opiates and NMBAs (compounds structurally related to opiates) [Moneret-Vautrin DA. et al., Anaphylaxis to general anesthesis. Chem Immunol Allergy 2010; 95:180-9] therefore the prior diagnosis of an alleged hypersensitivity to same can save a patient's life.

Nevertheless, performing an etiological allergological diagnosis on an anesthetized patient is very difficult because the skin reactions are concealed under surgical sheets, the patient cannot complain of irritation or dyspnea since he/she is anesthetized, and furthermore, there are many drugs used. Likewise, any method for preventing adverse events caused by drugs as typically used as opiates would constitute a very significant advantage in preventing adverse reactions to said drugs and would have a great health and social repercussion.

Skin tests (e.g., Prick Test and intradermoreaction), are *in vivo* tests which are one of the diagnostic methods used for confirming the clinical suspicions of allergic reactions to NMBAs. However, skin prick testing has not been shown to have diagnostic value when opiate reagents, such as morphine (10 mg/mL), meperidine or pethidine (50 mg/mL) and papaveretum [a mixture of opium alkaloid hydrochlorides made up of 253 parts of morphine hydrochloride, 23 parts of papaverine hydrochloride and 20 parts of codeine hydrochloride (British Pharmacopeia - 1993)] (15.4 mg/mL) are used and are assayed at 4 dilutions at a ratio of 1:10 [Nasser SM. et al., Opiate-sensitivity: clinical characteristics and the role of skin prick testing. Clin Exp Allergy 2001; 31(7):1014-20].

*In vitro* methods based primarily on NMBA-specific serum immunoglobulin E (IgE) quantification constitute another diagnostic alternative typically used for evaluating allergy or hypersensitivity to NMBAs. These *in vitro* tests have clearly shown the presence of serum IgE specific to alcuronium, d-tubocurarine, pancuronium and analogues, vecuronium, succinylcholine, decamethonium and gallamine in patients allergic to these NMBAs [Baldo BA. et al., In vitro diagnosis and studies on the mechanism(s) of anaphylactoid reactions to muscle relaxant drugs. Ann Fr Anesth Reanim 1985; 4(2):139-45].

Although various methods have been described for diagnosing whether a subject has an allergy or hypersensitivity to opiates and NMBAs used in anesthesia, there is still a need to develop alternative methods which allow diagnosing whether a subject has an allergy or hypersensitivity to opiates and/or to structurally related compounds used in anesthesia in a manner that is objective, safe for the patients, simple and low cost. Advantageously, said diagnostic method must also be highly sensitive and specific as well as have a high negative predictive value.

### Brief Description of the Invention

The authors of the present invention have found that a *Papaver somniferum* (hereinafter *P*. *somniferum*) seed extract can be used for diagnosing whether a subject has an allergy or hypersensitivity to opiates or to compounds structurally related to opiates, particularly opiates and NMBAs used in anesthesia. In a particular embodiment, said *P. somniferum* seed extract is a water soluble extract, whereas in another particular embodiment, said *P. somniferum* seed extract is a fat soluble extract; likewise, in another particular embodiment, said *P. somniferum* seed extract is a mixture of a water soluble and a fat soluble *P. somniferum* seed extract.

The experimental assays conducted have clearly shown that the serum of patients allergic to opiates contained antibodies (specific IgE) against said extracts (i.e., against components present in said extracts) therefore said *P*. *somniferum* seed extracts, both the water soluble extracts and the fat soluble extracts, alone or combined with one another, can be used in diagnosing the allergy or hypersensitivity to opiates, particularly opiates used in anesthesia.

Furthermore, it has been observed that said *P*. *somniferum* seed extracts, both the water soluble extracts and the fat soluble extracts, alone or combined with one another, can be used for measuring the allergy or hypersensitivity to NMBAs indirectly, therefore they can also be used in diagnosing allergy or hypersensitivity to said NMBAs.

Therefore, in a first aspect, the invention relates to the use of a *P*. *somniferum* seed extract in the preparation of a reagent for diagnosing allergy or hypersensitivity to an opiate or to a compound structurally related to an opiate.

In another aspect, the invention relates to methods for producing water soluble and fat soluble *P*. *somniferum* seed extracts as defined in claims 6 to 9.

The invention also relates to a *P*. *somniferum* seed extract obtainable according to any of the methods described above.

Another aspect of the invention relates to an *in vitro* method for diagnosing whether a subject has an allergy or hypersensitivity to an opiate or to a compound structurally related to an opiate which comprises:
a)contacting a biological sample from said subject susceptible of containing antibodies with an extract according to any of the extracts defined above; and
b)analyzing whether an interaction occurred between an IgE specific to said opiate or compound structurally related to an opiate possibly present in said biological sample susceptible of containing antibodies and said extract;
wherein an interaction between said IgE specific to said opiate and said extract is indicative that said subject has an allergy or hypersensitivity to said opiate.

An additional aspect of the invention relates to a kit comprising an extract according to any of the extracts described above.

A final aspect of the invention relates to the use of a kit as defined above for diagnosing whether a subject has an allergy or hypersensitivity to an opiate or to a compound structurally related to an opiate.

### Brief Description of the Drawings

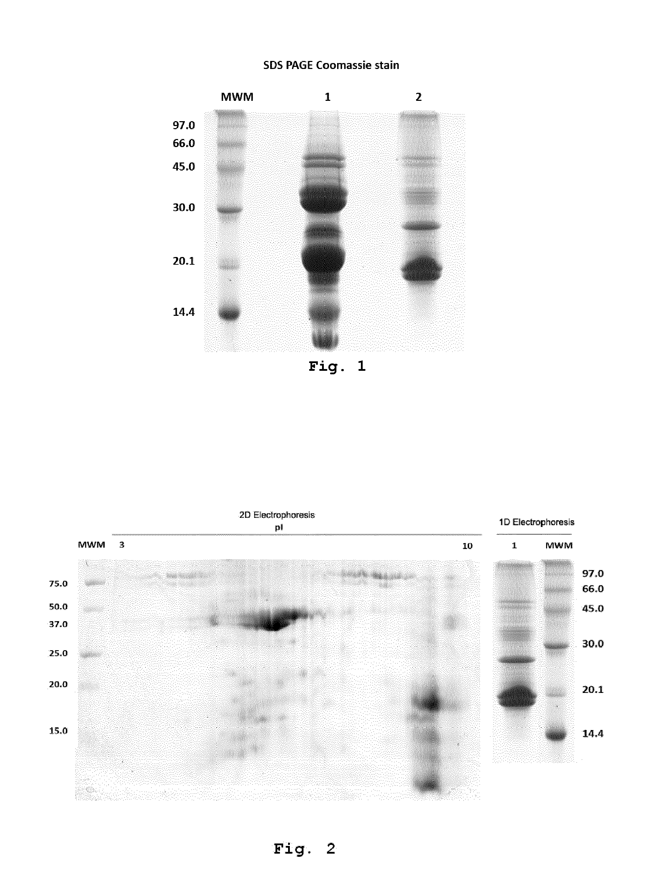

Figure 1 shows the electrophoretic profile of the water soluble and fat soluble *P. somniferum* seed extracts produced according to the method described in Example 1 determined by means of 15% SDS-PAGE and Coomassie blue stain. [MWM: molecular weight marker. Lane 1: water soluble extract produced by means of the production method [1] for producing a water soluble extract of the invention. Lane 2: fat soluble extract produced by means of the production method [A] for producing a fat soluble extract of the invention].
Figure 2 shows the electrophoretic profile of the fat soluble *P*. *somniferum* seed extract produced according to the method described in Example 1 (by means of the production method [A] for producing a fat soluble extract of the invention), determined by means of 15% one-dimensional SDS-PAGE (right panel - 1D Electrophoresis) and 15% two-dimensional SDS-PAGE [2D-15% SDS-PAGE] (left panel - 2D Electrophoresis) and with Coomassie blue stain in both cases. [MWM: Molecular weight marker].

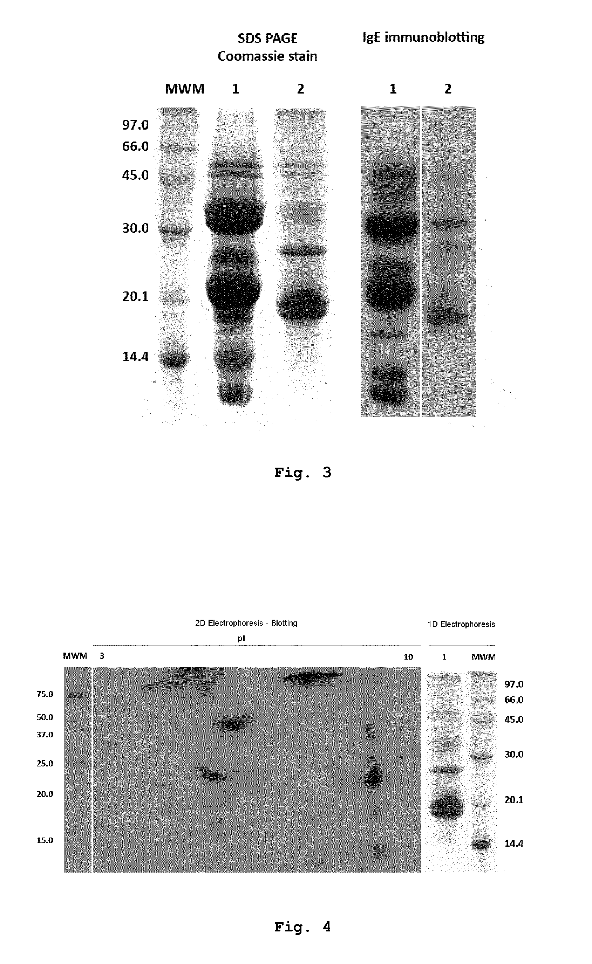

Figure 3 shows the allergenic profile of the water soluble and fat soluble *P*. *somniferum* seed extracts produced according to the method described in Example 1 determined by means of 15% SDS-PAGE and Coomassie blue stain and IgE immunoblotting. [MWM: molecular weight marker. Lane 1: water soluble extract. Lane 2: fat soluble extract. IgE: Immunoblotting with sensitized patient serum pool].
Figure 4 shows the allergenic profile of the fat soluble *P*. *somniferum* seed extract produced according to the method described in Example 1, determined by means of 15% two-dimensional SDS-PAGE [2D-15% SDS-PAGE] (left panel - 2D Electrophoresis) and Coomassie blue stain, and IgE immunoblotting. Furthermore, the right panel (1D Electrophoresis) includes the electrophoretic profile of said fat soluble extract determined by means of 15% one-dimensional SDS-PAGE and Coomassie blue stain. [MWM: molecular weight marker. pI: Isoelectric point. Lane 1 (1D Electrophoresis): fat soluble extract].

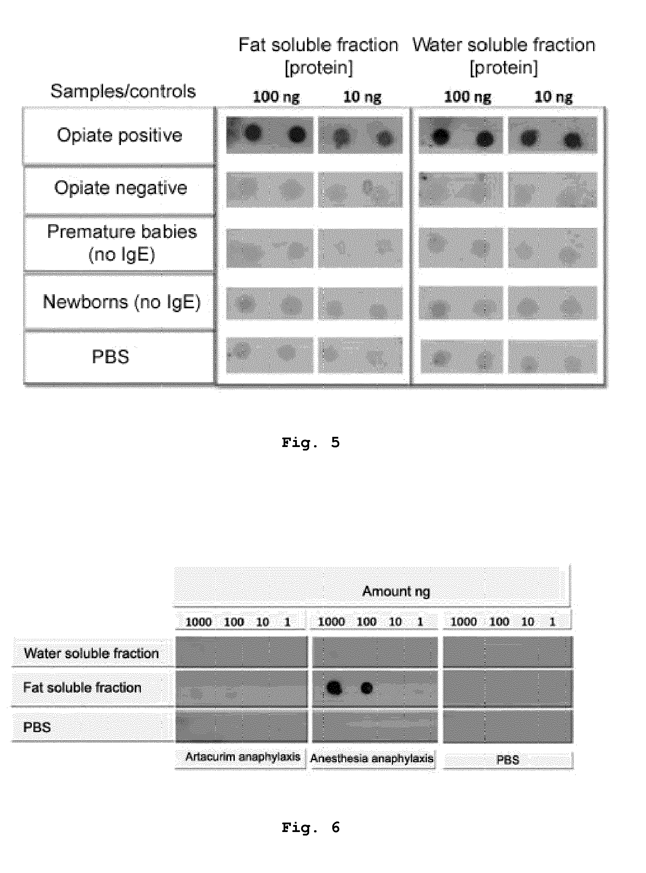

Figure 5 shows the results of a Dot Blot for detecting opiate-specific IgE in (i) the sera from 6 patients allergic to morphine derivatives (opiate positive), (ii) sera from patients not allergic to morphine derivatives (opiate negative); and sera from premature babies, sera from newborns and PBS buffer (controls), using the water soluble and fat soluble *P*. *somniferum* seed extracts produced according to the method described in Example 1.
Figure 6 shows the results of a Dot Blot for detecting opiate-specific IgE in (i) serum from a patient with a serious allergic reaction to the application of atracurium (diluted to 1:4 in blocking buffer (0.5% PBS Tween)); and (ii) a mixture of sera from 4 patients with a serious clinical reaction due to the application of anesthesia: fentanyl, alfentanil, morphine, propofol, droperidol, thiopental and/or ketamine, using the water soluble and fat soluble *P*. *somniferum* seed extracts produced according to the method described in Example 1. PBS was used as control.

### Detailed Description of the Invention

A first aspect of the invention is the use of a *P*. *somniferum* seed extract for the preparation of a reagent for diagnosing allergy or hypersensitivity to an opiate or to a compound structurally related to an opiate.

In a particular embodiment, said *P*. *somniferum* seed extract is a water soluble extract, whereas in another particular embodiment, said *P. somniferum* seed extract is a fat soluble extract; likewise, in another particular embodiment, said *P. somniferum* seed extract is a mixture of a water soluble and a fat soluble *P. somniferum* seed extract.

### Extract of the invention

In an additional aspect, the invention relates to a *P*. *somniferum* seed extract which can be produced by means of methods like those which will be described below, hereinafter said extract is referred to as extract of the invention.

In a particular embodiment, the extract of the invention is a water soluble extract, whereas in another particular embodiment, the extract of the invention is a fat soluble extract. Example 1 describes a particular embodiment of a water soluble and a fat soluble *P. somniferum* seed extract. Poppy (*Papaver somniferum* L.) is one of the most important cultivated plants for the pharmaceutical industry since it is the only source of alkaloids such as morphine, codeine and thebaine which are widely used in medicine as analgesics, anesthetics, antitussives and anticonvulsants. Poppy is an annual herbaceous plant, the fruits of which are flattened globular capsules ending with a lobed stigmatic crown containing numerous small reniform seeds having a reticulated surface which are white to dark brown in color. Capsule maturation coincides with a high concentration of alkaloids.

The water soluble *P. somniferum* seed extract can be produced by means of any of methods [1] and [2] described below.

In a particular embodiment, the invention provides a method for producing a water soluble *P. somniferum* seed extract, hereinafter "production method [1] for producing a water soluble extract of the invention", comprising the steps of:
a) suspending *P. somniferum* seeds in a medium comprising a buffer with a pH comprised between approximately 6 and approximately 9 and glycerol;
b) subjecting the *P. somniferum* seeds contained in the suspension obtained in step a) to a homogenization treatment at a temperature comprised between approximately 0°C and approximately 10°C for obtaining a *P. somniferum* seed homogenate comprising (i) a solid phase comprising *P*. *somniferum* seed cell debris and (ii) a liquid phase comprising the substances contained in said seeds;
c) subjecting the homogenate obtained in step b) to stirring at a temperature comprised between approximately 0°C and approximately 10°C;
d) separating the solid and liquid phases of the homogenate resulting from step c);
e) centrifuging the liquid phase obtained in step d) between 7,000 and 13,000 g for a time period sufficient to separate a liquid aqueous phase comprising a water soluble *P. somniferum* seed extract and a solid or semi-solid oily phase comprising a fat soluble *P. somniferum* seed extract formed by the substances contained in the *P. somniferum* seeds which are not soluble in an aqueous medium;
f) separating the aqueous phase comprising a water soluble *P. somniferum* seed extract obtained in step e) ;
g) filtering said aqueous phase comprising a water soluble *P. somniferum* seed extract separated in step f) through a filter with a pore size comprised between approximately 0.8 micrometers (µm) and approximately 8 µm for producing a filtrate comprising a water soluble *P. somniferum* seed extract; and
h) subjecting said filtrate comprising a water soluble *P. somniferum* seed extract to dialysis against deionized water with membranes having a molecular weight cut-off comprised between approximately 3,500 Da and approximately 7,000 Da at a temperature comprised between approximately 0°C and approximately 10°C for producing a water soluble *P. somniferum* seed extract.

Briefly, the production method [1] for producing a water soluble extract of the invention allows producing a water soluble *P. somniferum* seed extract from a *P. somniferum* seed suspension in a medium comprising a buffer with a pH comprised between approximately 6 and approximately 9 and glycerol obtained by mixing said seeds with said buffer and glycerol [step a)]. Virtually any buffer with a pH comprised between approximately 6 and approximately 9 can be used; nevertheless, in a particular embodiment, said buffer is selected from maleate buffer (pH 6.2-6.8), phosphate buffer (pH 6.9-8.0) in its different forms (e.g., Sörensen, Maunsbach, Milloning) and Tris-HCl buffer (pH 7.2-7.7); in a specific embodiment, said buffer is phosphate buffered saline (PBS). The buffer:glycerol ratio (v/v) can vary within a range; nevertheless, in a particular embodiment said medium comprises between 20% and 80% (v/v) of glycerol; in a specific embodiment said medium comprises approximately 25% (v/v) of glycerol. In a particular embodiment, intact *P. somniferum* seeds, i.e., seeds that are not broken, crushed or ground, are used; nevertheless, in another particular and preferred embodiment, the seeds are subjected to a prior process of breaking, crushing and/or grinding by conventional methods, for example, by means of using a mortar and pestle, etc., for the purpose of facilitating the extraction of different water soluble or fat soluble components of the *P. somniferum* seeds.

The *P. somniferum* seed suspension obtained in step a) is kept at a temperature comprised between approximately 0°C and approximately 10°C, preferably between approximately 2°C and approximately 8°C, typically about 5°C, for a time period comprised between approximately 30 minutes and 2 hours [step b)] for the purpose of homogenizing the *P. somniferum* seed suspension and solubilizing the greatest possible amount of water soluble or fat soluble substances contained in said *P. somniferum* seeds. The *P. somniferum* seeds contained in the suspension obtained in step a) are subjected to a cell disruption process by means of homogenization to release the content thereof into the medium in which they were suspended. Said process is performed by conventional methods known by persons skilled in the art, for example, by the mechanical action of a mortar and a pestle, by the action of an electric homogenizer, etc. A *P. somniferum* seed homogenate comprising (i) a solid phase comprising *P. somniferum* seed cell debris and (ii) a liquid phase comprising the content of said seeds, for example, the water soluble or fat soluble substances present in said seeds, is obtained after homogenization.

The homogenate obtained in step b) comprising said solid and liquid phases is then subjected to stirring [step c)], preferably continuous and constant stirring (controlled stirring), for example by means of magnetic stirring, to facilitate solubilizing the (water soluble and fat soluble) components of said liquid phase in the mixture formed by buffer and glycerol. The stirring is carried out at a temperature comprised between approximately 0°C and approximately 10°C, preferably between approximately 2°C and approximately 8°C, typically about 5°C, for a time period comprised between approximately 30 minutes and approximately 2 hours, preferably about 30 minutes. A "stirred" homogenate comprising (i) a solid phase comprising insoluble debris of said seeds and other solid components, and (ii) a liquid phase comprising the water soluble and fat soluble substances of the *P*. *somniferum* seeds, is obtained after stirring. Said liquid phase is then separated from said solid phase [step d)] by conventional solid and liquid separation methods, for example, by means of decantation, sieving, filtration, etc., for the purpose of obtaining the cleanest possible homogenate upon removing the solid phase comprising the insoluble *P*. *somniferum* seed debris. In a particular embodiment, said liquid phase is separated from said solid phase by sieving using a sieve with a mesh size suitable for removing the cell debris and fractions of seeds broken during homogenization; in a specific embodiment, said sieve has a mesh size of approximately 400 µm; nevertheless, sieves with a smaller mesh size can be used.

The separated liquid phase is then centrifuged [step e)] between approximately 7,000 g and approximately 13,000 g, preferably about 10,000 g, for a time period sufficient to separate a liquid aqueous phase comprising a water soluble *P*. *somniferum* seed extract and a solid or semi-solid oily phase comprising a fat soluble *P*. *somniferum* seed extract, generally comprised between approximately 5 and 30 minutes, preferably for about 15 minutes. This centrifugation is generally carried out at a temperature comprised between approximately 0°C and approximately 10°C, preferably between approximately 2°C and approximately 8°C, typically about 5°C. The non-fatty (water soluble) content is separated from the fatty (fat soluble) content under the indicated conditions after centrifugation; the fatty content (oily phase) is generally in a solid or semi-solid state and comprises the substances contained in *P*. *somniferum* seeds which are not soluble in an aqueous medium whereas the non-fatty content (aqueous phase) is in a liquid state and comprises the substances contained in *P*. *somniferum* seeds which are soluble in an aqueous medium.

The aqueous (liquid) phase obtained in step e) is separated [step f)] from the oily (solid or semi-solid) phase by conventional methods for separating liquid phases from solid or semi-solid phases known by persons skilled in the art; in a particular embodiment, said separation is performed by means of decantation, for which the oily phase present in the solid or semi-solid state (always in the upper part of the sample) is removed by means of using a spatula or a similar tool, attempting to collect the greatest possible amount of oily phase.

The aqueous phase comprising a water soluble *P*. *somniferum* seed extract is filtered [step g)] through a filter with a pore size comprised between approximately 0.8 µm and approximately 8 µm, preferably about 2 µm, for the purpose of removing impurities and obtaining a filtrate comprising a water soluble *P. somniferum* seed extract. Virtually any filter with said pore size can be used for putting this invention into practice; nevertheless, in a particular embodiment, said filter comprises a membrane with a glass fiber prefilter for protecting the membrane, such as a filter from the AP series (Millipore®), for example, from the AP 15 series (glass fiber filter with a membrane having a pore size comprised between 0.2 and 0.6 µm), AP 20 series (glass fiber filter with a membrane having a pore size comprised between 0.8 and 8 µm) or AP 25 series (glass fiber filter with a membrane having a pore size comprised between 0.9 and 8 µm).

The filtrate comprising the water soluble *P*. *somniferum* seed extract obtained in step g) is then subjected to dialysis [step h)] against deionized water with membranes having a molecular weight cut-off comprised between approximately 3,500 Da and approximately 7,000 Da, preferably about 3,500 Da, at a temperature comprised between approximately 0°C and approximately 10°C, preferably between approximately 2°C and approximately 8°C, typically about 5°C, for producing a water soluble *P*. *somniferum* seed extract. The duration of this dialysis treatment depends to a certain extent on the dialyzer apparatus used and it is generally comprised between approximately 1 hour (when the exchange flow is high and it is a cassette with a cut-off in the indicated range) and approximately 16 hours (when the membrane used is a "tripe"-type membrane).

The water soluble *P*. *somniferum* seed extract which can be produced according to the production method [1] for producing a water soluble extract of the invention described above is an additional aspect of the invention. The term "water soluble" is applied to the soluble substances [very soluble, freely soluble, soluble, sparingly soluble or slightly soluble (Spanish Pharmacopeia)] in water or in an aqueous medium. As used herein, a "water soluble *P*. *somniferum* seed extract" refers to a composition of matter comprising a substance or a group of substances obtained by extraction from *P*. *somniferum* seeds using water or an aqueous medium. Example 1 describes the production of a water soluble *P*. *somniferum* seed extract provided by this invention and its electrophoretic profile, wherein the existence of a band of about 52 kDa which may correspond to a major protein, as well as bands of about 20 kDa and 32 kDa which seem to be important specific IgE-binding proteins in the sera of the analyzed patients (Figure 3), can be seen.

Alternatively, the water soluble *P*. *somniferum* seed extract of the invention can be produced by means of a method for producing a water soluble *P*. *somniferum* seed extract, hereinafter "production method [2] for producing a water soluble extract of the invention", comprising the steps of:
a) contacting *P*. *somniferum* seeds with an organic polar aprotic solvent at a temperature comprised between approximately 0°C and approximately 10°C for obtaining a *P*. *somniferum* seed homogenate comprising a solid phase comprising insoluble *P*. *somniferum* seed debris and a liquid phase comprising said organic polar aprotic solvent and substances originating from said seeds soluble in said organic polar aprotic solvent;
b) separating said liquid phase generated in step a) under conditions which allow leaving the solid phase of step a) substantially free of said liquid phase;
c) contacting said solid phase substantially free of liquid phase of step b) with a medium comprising (i) a buffer with a pH comprised between approximately 6 and approximately 9 and (ii) a chelating agent, at a temperature comprised between approximately 0°C and approximately 10°C for extracting the water soluble fraction from the *P*. *somniferum* seeds and generating a suspension comprising a water soluble *P*. *somniferum* seed extract;
d) centrifuging the suspension obtained in step c) between approximately 6,000 g and approximately 12,000 g for a time period comprised between approximately 15 minutes and approximately 2 hours at a temperature comprised between approximately 0°C and approximately 10°C and separating the supernatant comprising a water soluble *P*. *somniferum* seed extract;
e) filtering the supernatant of step d) comprising a water soluble *P*. *somniferum* seed extract through a filter with a pore size comprised between approximately 0.8 µm and approximately 8 µm for obtaining a filtrate comprising a water soluble *P*. *somniferum* seed extract; and
f) subjecting said filtrate comprising a water soluble *P. somniferum* seed extract obtained in step e) to dialysis against deionized water with membranes having a molecular weight cut-off comprised between approximately 3,500 Da and approximately 7,000 Da at a temperature comprised between approximately 0°C and approximately 10°C for producing a water soluble *P*. *somniferum* seed extract.

The production method [2] for producing a water soluble extract of the invention is an alternative method to the production method [1] for producing a water soluble extract of the invention for extracting the water soluble proteins (and other substances) contained in *P*. *somniferum* seeds, which allows producing a water soluble *P*. *somniferum* seed extract from said seeds. Briefly, said seeds (intact or crushed/ground) are contacted with an organic polar aprotic solvent [step a)]; illustrative examples of organic polar aprotic solvents include both organic polar aprotic solvents with high dielectric constants, for example, acetone, dimethylsulfoxide (DMSO), dimethylformamide (DMF), hexamethylphosphoramide (HMPA), nitriles (e.g., acetonitrile, butanonitrile, etc.), ketones (e.g., methylethylketone, butanone, etc.), and organic polar aprotic solvents with low dielectric constants, for example, tetrahydrofuran (THF), 1,4-dioxane, dichloromethane, ethyl acetate, 1,2-dimethoxyethane (monoglyme), etc.; in a particular and preferred embodiment, said organic polar aprotic solvent is acetone. Homogenization, i.e., the disruption of the cells of *P*. *somniferum* seeds and the release of their content into the medium in which they were suspended (organic polar aprotic solvent) takes place by means of this treatment; this treatment can be performed by conventional methods known by persons skilled in the art, for example, by the mechanical action of a mortar and a pestle, by the action of an electric homogenizer, etc. Said treatment is generally carried out at a temperature comprised between approximately 0°C and approximately 10°C, preferably between approximately 2°C and approximately 8°C, typically about 5°C. A *P*. *somniferum* seed homogenate comprising (i) a solid phase comprising insoluble *P*. *somniferum* seed cell debris and (ii) a liquid phase comprising the organic polar aprotic solvent and the substances originating from said soluble in said organic polar aprotic solvent is obtained after this treatment. As indicated above, *P. somniferum* seeds can be used intact, i.e., without being broken, crushed or ground, or, preferably, fragmented, crushed or ground for which purpose said seeds are subjected to a prior process of breaking, crushing and/or grinding by conventional methods, for example, by means of using a mortar and pestle, etc., for the purpose of facilitating the extraction of different substances from the *P*. *somniferum* seeds soluble in the organic polar aprotic solvent used.

The liquid (organic) phase obtained in step a) is then separated under conditions which allow leaving the solid phase of step a) substantially free of said liquid phase [step b)]. The removal or separation of said liquid phase can be carried out by conventional phase separation methods known by persons skilled in the art; in a non-limiting illustrative manner, said separation can be performed by means of filtration through membranes with a pore size comprised between approximately 0.8 µm and approximately 8 µm, for example, of about 2 µm, or by means of evaporation, using for example a rotavapor system (as is known, the boiling point of acetone at 1 atm is 56°C), etc.

The solid phase originating from step b) is then contacted with a medium comprising a buffer with a pH comprised between approximately 6 and approximately 9 and glycerol obtained by mixing said seeds with said buffer and a chelating agent for extracting the water soluble fraction from the *P*. *somniferum* seeds and generating a suspension comprising a water soluble extract of said seeds [step c)]. Virtually any buffer with a pH comprised between approximately 6 and approximately 9 can be used; nevertheless, in a particular embodiment, said buffer is selected from maleate buffer (pH 6.2-6.8), phosphate buffer (pH 6.9-8.0) in its different forms (e.g., Sörensen, Maunsbach, Milloning) and Tris-HCl buffer (pH 7.2-7.7); in a specific embodiment, said buffer is Tris-HCl (pH 7.5). The chelating or sequestering agent generally prevents the formation of insoluble compounds and therefore the action of undesired effects when forming stable complexes with the metal ions. Virtually any chelating agent can be used in putting this invention into practice; nevertheless, in a particular embodiment, said chelating agent is selected from the group consisting of ethylenediaminetetraacetic acid (EDTA), diethylenetriaminepentaacetic acid (DTPA), nitrilotriacetic acid (NTA), tripyrrolidinophosphoric acid triamide (TPPA), gluconic acid, citric acid, tartaric acid, malic acid, lactic acid, acetic acid and mixtures thereof, preferably EDTA, a chelating agent containing amino carboxyl groups with a strong chelating power. The (solid phase originating from step b)):(buffer-chelating agent) ratio can vary within a range; nevertheless, in a particular embodiment said ratio is comprised between approximately 1:0.1 and approximately 1:25 (w/v), typically, at a ratio comprised between 1:1 and 1:10 (w/v); in a particular ratio, said (solid phase originating from step b)):(buffer-chelating agent) ratio is 1:5 (w/v). This step is carried out at a temperature comprised between approximately 0°C and approximately 10°C, preferably between approximately 2°C and approximately 8°C, typically about 5°C for a time period comprised between approximately 15 minutes and approximately 2 hours, generally between 30 minutes and 1 hour. A suspension comprising a water soluble *P*. *somniferum* seed extract is obtained after this treatment.

The resulting suspension after the extraction of the water soluble substances from said seeds is centrifuged [step d)] between approximately 6,000 g and approximately 12,000 g, preferably at approximately 9,000 g for a time period comprised between approximately 15 minutes and approximately 2 hours, generally between 30 minutes and 1 hour, at a temperature comprised between approximately 0°C and approximately 10°C, preferably between approximately 2°C and approximately 8°C, typically about 5°C, for the purpose of separating the supernatant comprising a water soluble *P*. *somniferum* seed extract which is filtered [step e)] through a filter with a pore size comprised between approximately 0.8 µm and approximately 8 µm, preferably about 2 µm, for the purpose of removing impurities and obtaining a filtrate comprising a water soluble *P. somniferum* seed extract. Virtually any filter with said pore size can be used; nevertheless, in a particular embodiment, said filter is a glass fiber filter of the AP series (Millipore®) comprising a membrane with a pore size comprised between 0.2 and 0.6 µm (AP 15 series), or a membrane with a pore size comprised between 0.8 and 8 µm (AP 20) or a membrane with a pore size comprised between 0.9 and 8 µm (AP 25).

The filtrate comprising the water soluble *P*. *somniferum* seed extract obtained in step e) is then subjected to dialysis [step f)] against deionized water with membranes having a molecular weight cut-off comprised between approximately 3,500 Da and approximately 7,000 Da, preferably about 3,500 Da, at a temperature comprised between approximately 0°C and approximately 10°C, preferably between approximately 2°C and approximately 8°C, typically about 5°C, for producing a water soluble *P*. *somniferum* seed extract. The duration of this dialysis treatment depends to a certain extent on the dialyzer apparatus used and generally it is comprised between approximately 1 hour (when the exchange flow is high and it is a cassette with a cut-off in the indicated range) and approximately 16 hours (when the membrane used is a "tripe"-type membrane).

The water soluble *P*. *somniferum* seed extract which can be produced according to the production method [2] for producing a water soluble extract of the invention described above is an additional aspect of the invention.

The invention also provides a fat soluble *P*. *somniferum* seed extract. Said fat soluble *P*. *somniferum* seed extract can be produced by means of a method such as that described below.

In a particular embodiment, the invention provides a method for producing a fat soluble *P*. *somniferum* seed extract, hereinafter "production method [A] for producing a fat soluble extract of the invention", comprising the steps of:
a) suspending *P*. *somniferum* seeds in a medium comprising a buffer with a pH comprised between approximately 6 and approximately 9 and glycerol;
b) subjecting the *P*. *somniferum* seeds contained in the suspension obtained in step a) to a homogenization treatment at a temperature comprised between approximately 0°C and approximately 10°C for obtaining a *P*. *somniferum* seed homogenate comprising (i) a solid phase comprising *P*. *somniferum* seed cell debris and (ii) a liquid phase comprising the substances contained in said seeds;
c) subjecting the homogenate obtained in step b) to stirring at a temperature comprised between approximately 0°C and approximately 10°C;
d) separating the solid and liquid phases of the homogenate resulting from step c);
e) centrifuging the liquid phase obtained in step d) between 7,000 and 13,000 g for a time period sufficient to separate a liquid aqueous phase comprising a water soluble *P*. *somniferum* seed extract and a solid or semi-solid oily phase comprising a fat soluble *P*. *somniferum* seed extract formed by the substances contained in the *P*. *somniferum* seeds which are not soluble in an aqueous medium;
f) separating the oily phase comprising a fat soluble *P*. *somniferum* seed extract obtained in step e);
g) resuspending the oily phase originating from step f) in a medium comprising (i) a buffer with a pH comprised between approximately 6 and approximately 9 and (ii) glycerol;
h) centrifuging the suspension obtained in step g) between approximately 7,000 g and approximately 13,000 g for a time period comprised between approximately 2 minutes and approximately 15 minutes for obtaining an oily phase comprising a fat soluble *P. somniferum* seed extract;
i) resuspending the oily phase originating from step h) in a medium comprising (i) a buffer with a pH comprised between approximately 6 and approximately 9 and (ii) glycerol;
j) centrifuging the suspension obtained in step i) between approximately 7,000 g and approximately 13,000 g for a time period comprised between approximately 2 minutes and approximately 15 minutes for obtaining an oily phase comprising a fat soluble *P. somniferum* seed extract;
k) separating the oily phase originating from step j);
l) resuspending the oily phase originating from step k) with a medium comprising at least one organic solvent;
m) centrifuging the suspension obtained in step l) between approximately 2,000 g and approximately 5,000 g for a time period comprised between approximately 5 minutes and approximately 15 minutes for obtaining an oily phase comprising a fat soluble *P*. *somniferum* seed extract;
n) separating the oily phase originating from step m);
o) adding an organic solvent to the oily phase originating from step n) at a temperature comprised between approximately -20°C and approximately 10°C and maintaining at a temperature comprised between approximately -20°C and approximately 10°C for a time period comprised between approximately 5 minutes and approximately 25 minutes; and
p) centrifuging the suspension resulting from step o) between approximately 7,000 g and approximately 10,000 g, preferably about 10,000 g, for a time period comprised between approximately 5 minutes and approximately 30 minutes for obtaining a fat soluble *P. somniferum* seed extract.

Steps a) - e) of the production method [A] for producing a fat soluble extract of the invention are the same as steps a) - e) of the production method [1] for producing a water soluble extract of the invention, the features of which have already been mentioned above.

In step f) of the production method [A] for producing a fat soluble extract of the invention, the oily phase comprising a fat soluble *P*. *somniferum* seed extract is separated and resuspended in a medium comprising a buffer with a pH comprised between approximately 6 and approximately 9 and glycerol obtained by mixing said seeds with said buffer and glycerol. Virtually any buffer with a pH comprised between approximately 6 and approximately 9 can be used; nevertheless, in a particular embodiment, said buffer is selected from maleate buffer (pH 6.2-6.8), phosphate buffer (pH 6.9-8.0) in its different forms (e.g., Sörensen, Maunsbach, Milloning) and Tris-HCl buffer (pH 7.2-7.7); in a specific embodiment, said buffer is a pH 7.4 phosphate buffered saline (PBS). The buffer:glycerol ratio (v/v) can vary within a range; nevertheless, in a particular embodiment said medium comprises between 20% and 80% (v/v) of glycerol; in a specific embodiment said medium comprises approximately 50% (v/v) of glycerol.

The suspension obtained above is centrifuged [step h)] between approximately 7,000 g and approximately 13,000 g, preferably at approximately 10,000 g, for a time period comprised between approximately 2 minutes and approximately 15 minutes, generally between 2.5 minutes and 10 minutes, typically about 5 minutes. The centrifugation can be performed at a temperature comprised between approximately 0°C and approximately 10°C, preferably between approximately 2°C and approximately 8°C, typically about 5°C. The oily phase which has been resuspended in the medium comprising the buffer and glycerol is washed by means of this treatment for the purpose of removing possible protein residues or other water soluble substances which may have remained in the preceding step, whereby a new oily phase in solid or semi-solid state which would include the substances and proteins forming part of the lipid bodies present in *P*. *somniferum* seeds is obtained. Said oily phase (solid or semi-solid) is separated by conventional methods for separating liquid phases from solid or semi-solid phases known by persons skilled in the art, for example, by means of decantation using a spatula or a similar tool, as mentioned above.

The oily phase originating from step h) is resuspended [step i)] in a medium comprising (i) a buffer with a pH comprised between approximately 6 and approximately 9 and (ii) glycerol, as mentioned above in relation to the preceding step g) and the resulting suspension is centrifuged [step j)] in conditions similar to those described in relation to step h); i.e., between approximately 7,000 g and approximately 13,000 g, preferably at approximately 10,000 g, for a time period comprised between approximately 2 minutes and approximately 15 minutes, generally between 2.5 minutes and 10 minutes, typically about 5 minutes. The centrifugation can be performed at a temperature comprised between approximately 0°C and approximately 10°C, preferably between approximately 2°C and approximately 8°C, typically about 5°C. The oily phase which has been resuspended in the medium comprising the buffer and glycerol is washed again by means of this treatment for the purpose of removing possible protein residues or other water soluble substances which may have remained in the preceding step, whereby a new oily phase in solid or semi-solid state which would include the substances and proteins forming part of the lipid bodies present in *P*. *somniferum* seeds is obtained. If desired, in a particular embodiment, after washing with buffer and glycerol, an additional washing of the oily phase can be performed with a buffer with a pH close to 10, for example, with a pH comprised between 9 and 11, preferably between approximately 9.5 and approximately 10.5 for example, with a pH 9.6 carbonate/bicarbonate buffer. Said oily phase (solid or semi-solid) is separated [step k)] by conventional methods for separating liquid phases from solid or semi-solid phases known by persons skilled in the art, for example, by means of decantation using a spatula or a similar tool, as mentioned above, and is resuspended in a medium comprising one or more organic solvents [step l)], for example, an organic protic or aprotic polar solvent for example, methanol, ethanol, acetone, acetic acid, THF, etc., or a non-polar organic solvent, for example, diethylether, chloroform, benzene, toluene, xylene, ketones (other than acetone), hexane, cyclohexane, carbon tetrachloride, etc. In a particular embodiment, said medium comprises a chloroform:methanol mixture; the ratio between chloroform and methanol present in said mixture can vary within a wide range, for example, said mixture can contain between 20% and 60% by volume of methanol; in a particular embodiment, said medium comprises a chloroform:methanol mixture at a ratio of 2:1 (v/v). The separation of the fat soluble proteins contained in the lipid bodies (probably those of greater interest for diagnosing allergy or hypersensitivity to opiates used in anesthesia) is favored by means of this treatment.

The suspension thus obtained in step l) is centrifuged between approximately 2,000 g and approximately 5,000 g, preferably about 3,000 g, for a time period comprised between approximately 5 minutes and approximately 15 minutes, preferably 10 minutes [step m)], for removing possible water soluble substances and proteins which may have been retained and obtaining an oily phase comprising a fat soluble *P*. *somniferum* seed extract increasingly free of water soluble substances and proteins, which is separated [step n)] by conventional methods as mentioned above in relation to step k) and to which an organic solvent is added at a temperature comprised between approximately -20°C and approximately 10°C [step o)]. Illustrative non-limiting examples of said organic solvent include acetone, ethyl acetate, THF, etc. In a particular embodiment, this step o) is carried out using "cold" acetone, i.e., at a temperature comprised between approximately -20°C and approximately 10°C, typically between approximately -10°C and approximately 5°C. The oily phase:acetone ratio can vary within a wide range, nevertheless, in a particular embodiment said ratio is comprised between approximately 1:2 (v/v) and approximately 1:4 (v/v), typically about 1:2 (v/v). The resulting suspension is left at a temperature comprised between approximately -20°C and approximately 10°C, typically between approximately -10°C and approximately 5°C, for a time period comprised between approximately 5 minutes and approximately 25 minutes, typically approximately about 15 minutes.

Finally, the suspension resulting from step o) is centrifuged [step p)] between approximately 7,000 g and approximately 10,000 g, preferably about 10,000 g, for a time period comprised between approximately 5 minutes and approximately 30 minutes, preferably between 10 and 20 minutes approximately, generally about 15 minutes, for producing a fat soluble *P*. *somniferum* seed extract.

The fat soluble *P*. *somniferum* seed extract which can be produced according to the production method [A] for producing a fat soluble extract of the invention described above is an additional aspect of the invention. As used herein, a "fat soluble *P*. *somniferum* seed extract" refers to a composition of matter comprising a substance or group of substances obtained from *P*. *somniferum* seeds, substantially free of water soluble substances and proteins (which have been/can be extracted by means of using aqueous media, e.g., buffers, etc.), and concentrated by means of using organic solvents; in a particular embodiment, said fat soluble *P*. *somniferum* seed extract comprises the oily fraction rich in proteins derived from the lipid bodies present in *P*. *somniferum* seeds, substantially free of water soluble substances and proteins obtained by means of using aqueous media, and concentrated by means of using organic solvents. As is known, lipid or oleic bodies are the intracellular lipid reserve compartments of organisms and although they perform multiple varied functions within the organism, mature lipid or oleic bodies share a simple structure comprising a hydrophobic core formed by a lipid reserve structure (e.g., triacylglycerides, cholesterol esters, etc.), and a single layer phospholipid coating with multiple associated proteins anchored to the surface. Some plants use the lipid bodies contained in the cells of the seeds as an energy source for germination. Example 1 describes the production of a fat soluble *P*. *somniferum* seed extract provided by this invention and its electrophoretic profile, wherein the following can be seen: a band with a relative molecular weight of about 16 kDa correspondingArgJ protein (involved in the biosynthesis of arginine), band(s) with a molecular weight of about 18-20 kDa which may correspond, in terms of relative molecular weight, with the so-called oleosins, a band with a relative molecular weight of about 23 kDa corresponding with an aquaporin, a band with a relative molecular weight of about 28 kDa corresponding with a phytochrome, a band with a relative molecular weight of about 34 kDa corresponding with a caleosin and bands with a relative molecular weight around 40 kDa which would correspond with the isoforms of 11S globulin protein.

The fat soluble *P*. *somniferum* seed extract of the invention can alternatively be produced by means of a method for producing a fat soluble *P*. *somniferum* seed extract, hereinafter "production method [B] for producing a fat soluble extract of the invention", comprising the steps of:
a) resuspending the oily phase originating from step f) of the production method [A] for producing a fat soluble extract of the invention in cold acetone at a temperature comprised between approximately -20°C and approximately 10°C and maintaining at a temperature comprised between approximately -20°C and approximately 10°C for a time period comprised between approximately 5 minutes and approximately 25 minutes; and
b) centrifuging the suspension resulting from step o) between approximately 7,000 g and approximately 10,000 g, preferably about 10,000 g, for a time period comprised between approximately 5 minutes and approximately 30 minutes for producing a fat soluble *P. somniferum* seed extract.

The production method [B] for producing a fat soluble extract of the invention is based on protein precipitation by means of the action of acetone. According to the production method [B] for producing a fat soluble extract of the invention, the oily phase originating from step f) of the production method [A] for producing a fat soluble extract of the invention is suspended in "cold" acetone [step a)], i.e., at a temperature comprised between approximately -20°C and approximately 10°C, typically between approximately -10°C and approximately 5°C. The oily phase:acetone ratio can vary within a wide range; nevertheless, in a particular embodiment said ratio is comprised between approximately 1:2 (v/v) and approximately 1:4 (v/v), typically about 1:2 (v/v). The resulting suspension is left at a temperature comprised between approximately -20°C and approximately 10°C, typically between approximately -10°C and approximately 5°C, for a time period comprised between approximately 5 minutes and approximately 25 minutes, typically approximately about 15 minutes.

Finally, the suspension resulting from step a) is centrifuged [step b)] between approximately 7,000 g and approximately 10,000 g, preferably about 10,000 g, for a time period comprised between approximately 5 minutes and approximately 30 minutes, preferably between approximately 10 and 20 minutes, generally about 15 minutes, for producing a fat soluble *P*. *somniferum* seed extract.

The fat soluble *P*. *somniferum* seed extract which can be produced according to the production method [B] for producing a fat soluble extract of the invention described above is an additional aspect of the invention.

The extracts of the invention, both the water soluble and the fat soluble *P*. *somniferum* seed extract, can be found in liquid or solid form before use. In a particular embodiment, said extracts are in liquid form, dissolved or suspended in a suitable medium such as, for example, a physiological saline solution, 50% glycerol saline solution, PBS buffer, etc., prepared for application or administration thereof to the subject. In the case of the fat soluble extract, it is advantageous to add a surfactant, preferably a polysorbate such as 1% Tween® 20 (v/v), for example, on the diluent (e.g., physiological saline solution, etc.) to solubilize it. In another particular embodiment, said reagents are in solid form, for example, in the form of a lyophilisate; in the latter case, said extracts can be lyophilized by conventional lyophilization methods known by persons skilled in the art in the presence of a cryoprotective agent, such as for example, a saccharide, (e.g., mannitol, etc.), and they are dissolved or suspended in a suitable medium, such as for example, a physiological saline, 50% glycerol saline solution, PBS, etc., before application or administration thereof to a subject and, in the case of the fat soluble extract a suitable surfactant, for example, a polysorbate such as Tween® 20 which solubilizes it would have to be added. In both particular embodiments, the final pH of the extracts is close to physiological pH, about 7.4 approximately. The extracts of the invention in the lyophilized form are an additional aspect of the present invention.

### Applications of the P. somniferum extracts

According to the present invention, and as mentioned above, both the water soluble and fat soluble *P*. *somniferum* seed extracts can be used in diagnosing allergy or hypersensitivity to opiates or compounds structurally related to said opiates.

In a preferred embodiment, the invention relates to the use of an extract of the invention in the preparation of a reagent for diagnosing allergy or hypersensitivity to an opiate or to a compound structurally related to an opiate. In a particular embodiment, the extract of the invention is selected from the group consisting of a water soluble *P*. *somniferum* seed extract provided by this invention, a fat soluble *P*. *somniferum* seed extract provided by this invention, and combinations of said extracts.

In the sense used in this description, the expression "allergy or hypersensitivity to an opiate or to a compound structurally related to an opiate" refers to the immediate induction of allergic symptoms due to the administration of one or more opiates or structurally related compounds through any route, particularly to a sensitized subject, i.e., a subject whose serum contains IgE antibodies specific to an opiate or to a compound structurally related to an opiate. Generally, the first phase in diagnosing allergy or hypersensitivity to an opiate or to a compound structurally related to an opiate comprises recording a clinical history of allergic symptoms to the opiate, and in a second phase, performing the confirmatory assays necessary for determining the alleged sensitization.

In the sense used in this description, the term "opiate" refers to an alkaloid extracted from poppy husk and its synthetic or semi-synthetic homologues which bind to the opioid receptors located mainly in the central nervous system (CNS) and the gastrointestinal tract. Although the term opiate is commonly used to refer to all opium like drugs, it is more suitable to limit its scope to natural opium alkaloids, for example, morphine (prototypic opiate), codeine, etc., and to their semi-synthetic derivatives, for example, heroin, oxycodone, etc., and synthetic derivatives, for example, pethidine, methadone, etc., having a structure not related to opium alkaloids. Opiates are used for their anesthetic, as well as analgesic and antitussive effects. Non-limiting illustrative examples of opiates include alfentanil, buprenorphine, carfentanil, codeine, dihydrocodeine, etorphine, fentanyl, heroin (diamorphine), hydrocodone, hydromorphone, loperamide, meperidine, methadone, morphine, oxycodone, oxymorphone, pethidine, pholcodine, remifentanil, sufentanil, tapentadol, tramadol, etc.

In a particular embodiment, said opiate is an opiate used in anesthesia; non-limiting illustrative examples of opiates used in anesthesia include fentanyl, meperidine, morphine, remifentanil, etc. In another particular embodiment, said opiate is an analgesic opiate, for example, morphine, codeine, heroin, methadone, meperidine, fentanyl, buprenorphine, pentazocine, naloxone, naltrexone, etc. In another particular embodiment, said opiate is an opiate used as an antitussive, for example, codeine, dihydrocodeine, noscapine, dextromethorphan, dimemorfan, etc. In another particular embodiment, said opiate is heroin.

As used herein, a "compound structurally related to an opiate" refers to a compound having a chemical structure similar to that of an opiate; in a particular embodiment, said compound structurally related to an opiate is a compound having a quaternary ammonium ion structure and containing a hydrophobic ring skeleton and a hydrophilic tertiary amine (i.e., a chemical structure similar to that of morphine); illustrative examples of compounds having that chemical structure similar to that of morphine include NMBAs. As used in the present description, the term "neuromuscular blocking agent" or (NMBA or NMBD - Neuromuscular Blocking Agent/Drug) refers to drugs blocking the neuromuscular transmission at the neuromuscular synapse level, causing paralysis of the affected skeletal muscle; neuromuscular block is used together with general anesthesia to induce paralysis for surgical purposes so that surgery, particularly abdominal and intrathoracic surgery, proceeds with fewer complications. These NMBAs generally have a chemical structure similar to that of morphine, based on a quaternary ammonium ion structure and contain a hydrophobic ring skeleton and a hydrophilic tertiary amine [Fischer MM. et al., Immunoassays for the diagnosis of anaphylaxis to neuromuscular blocking drugs: the value of morphine for the detection of IgE antibodies in allergic subjects. Anaesth Intensive Care 2000; 28:167-70]. Non-limiting illustrative examples of NMBAs include atracurium, cisatracurium, doxacurium, gallamine, glycopyrrolate, mivacurium, pancuronium, pipecuronium, rapacuronium, rocuronium, tubocurarine, vecuronium, etc. Although not intended to be linked by any theory, it is believed that the capacity of both the water soluble and the fat soluble *P. somniferum* seed extracts provided by this invention to indirectly evaluate the allergy or hypersensitivity to NMBAs can be due precisely due to the fact that said NMBAs share chemical structures similar to those of some opiates.

In a particular embodiment, said compound structurally related to an opiate is an NMBA as defined above, for example, atracurium, cisatracurium, doxacurium, gallamine, glycopyrrolate, mivacurium, pancuronium, pipecuronium, rapacuronium, rocuronium, tubocurarine, vecuronium, etc.

Said reagent for diagnosing allergy or hypersensitivity to an opiate or to a compound structurally related to an opiate comprises a *P*. *somniferum* seed extract, such as an extract of the invention, for example. In a particular embodiment, since the diagnostic tests can be performed *in vivo* in the subject him/herself, said reagent comprises, in addition to the extract, a pharmaceutically acceptable vehicle, i.e., a compound or combination of compounds compatible with the extract and not harmful for the subject; said pharmaceutically acceptable vehicles will generally be chosen depending on the way of administering the reagent comprising the extract to the subject, for example, by topical route or intradermal route in skin tests or by other routes, for example, in bronchial challenge tests, etc. A review of the different ways of administering a compound to a subject and of the different pharmaceutically acceptable vehicles for producing same can be found, for example, in the "Tratado de Farmacia Galénica", C. Fauli i Trillo, 1993, Luzán 5, S.A. Editions, Madrid; and in Remington's Pharmaceutical Sciences (A.R. Gennaro, Ed.), 20th edition, Williams & Wilkins PA, USA (2000).

The *P*. *somniferum* seed extract, such as for example, an extract of the invention, is particularly useful to know whether a subject has an allergy or hypersensitivity to opiates or to compounds structurally related to opiates.

As used herein, the term "subject" includes any mammal including human beings. Virtually any subject can be analyzed according to the present invention to know whether he/she has an allergy or hypersensitivity to an opiate. Nevertheless, in a particular embodiment, said subject is a subject in whom the existence of an allergy or hypersensitivity to opiates or to compounds structurally related to opiates is to be known, for example, if he/she has an allergy or hypersensitivity to an opiate used in anesthesia (this situation may arise, for example, in the event that said subject were to receive anesthesia for surgical purposes, etc.). In another particular embodiment, said subject is a subject suspected of having allergy or hypersensitivity to opiates, particularly to opiates used in anesthesia. In another particular embodiment, said subject is a subject in whom the existence of an allergy or hypersensitivity to opiates used as analgesics or antitussives is to be known (this situation may arise, for example, in the event that said subject were to receive an analgesic or antitussive drug the formulation of which will includes an opiate with analgesic or antitussive effects, etc.). In another particular embodiment, said subject is a subject who has had an immediate allergic reaction induced by the administration of one or more opiates, for example, one or more opiates used as anesthetic, analgesic or antitussive, whose skin tests with respect to a (water soluble and/or fat soluble) extract of the invention had been positive. In another particular embodiment, said subject is a subject in whom the existence of an allergy or hypersensitivity to a compound structurally related to an opiate, such as a NMBA, is to be known, for example, whether he/she has an allergy or hypersensitivity to an NMBA used in anesthesia (this situation may arise, for example, in the event that said subject were to receive anesthesia for surgical purposes, etc.). In another particular embodiment, said subject is a subject who has had an immediate allergic reaction induced by the administration of one or more NMBAs, for example, one or more NMBAs used in anesthesia, whose skin tests with respect to a (water soluble and/or fat soluble) extract of the invention had been positive. In a particular embodiment, said allergic reaction can include an anaphylactic reaction characterized by the presence of several of the following symptoms: systemic pruritus, maculopapular urticaria associated or not associated with the presence of angioedema in different parts of the body, dysphonia, breathing difficulty, abdominal cramps associated or not associated with hypotension, etc. In any case, the skilled person can assess the different reactions observed and according to his/her criterion, conduct an allergological test to confirm (or rule out) the suspicion of a possible allergy or hypersensitivity to said opiates or compounds structurally related to same (for example, NMBAs).

In a particular embodiment, diagnosing allergy or hypersensitivity to opiates or to compounds structurally related to opiates is carried out by means of an allergological study comprising performing *in vivo* diagnostic tests in a subject. Illustrative examples of said *in vivo* diagnostic tests include skin tests, such as the intraepidermal tests or SPT (skin prick tests), also known as "prick tests", and intradermal tests (ID) making up the main diagnostic methods used for confirming the clinical suspicions of allergic reactions, as well as challenge tests, for example, bronchial, nasal or eye challenge tests.

The skin tests (SPT and ID) are performed by means of conventional protocols. Briefly, a reagent comprising *P. somniferum* seed extract, such as an extract of the invention, for example, normally in the form of a solution or suspension, is applied or administered to the subject under study. The concentration of the extract in the reagent can vary within a wide range depending, among other factors, on the sensitivity of the subject to said opiates, in a particular embodiment, the concentration of the extract, expressed in unit of mass (protein), in mg, per unit of volume, in ml, to be used in the skin tests is comprised between approximately 0.01 and 10 mg/ml, generally between 0.05 and 5 mg/ml; in a particular embodiment, a set of extracts with protein concentrations of 1 mg/ml, 0.5 mg/ml, 0.1 mg/ml and 0.05 mg/ml for each fraction was prepared. In a particular embodiment, an SPT test or Prick Test is considered positive when the largest diameter of the papule obtained is equal to or greater than 3 mm upon applying the reagent comprising the extract to the subject and once the reading time (for example, 20 minutes) has lapsed and the response to the negative control (for example, 50% glycerol saline solution or diluent) is negative; likewise, in another particular embodiment, an ID test is considered positive when the difference between the size of the papule 20 minutes after applying the reagent comprising the extract and the size of the papule right after applying said extract is equal to or greater than 3 mm.

In another particular embodiment, diagnosing allergy or hypersensitivity to opiates or to compounds structurally related to opiates is carried out by means of an allergological study which comprises performing *in vitro* diagnostic tests. Illustrative examples of said *in vitro* diagnostic tests include methods for determining and quantifying specific serum IgE.

Therefore, in another aspect, the invention relates to an *in vitro* method for diagnosing whether a subject has an allergy or hypersensitivity to an opiate or to a compound structurally related to an opiate comprising:
a) contacting a biological sample from said subject susceptible of containing antibodies with a *P*. *somniferum* seed extract; and
b) analyzing whether an interaction occurred between an IgE specific to said opiate possibly present in said biological sample susceptible of containing antibodies and said extract;
wherein an interaction between said IgE specific to said opiate or compound structurally related to an opiate and the extract is indicative that said subject has an allergy or hypersensitivity to said opiate or compound structurally related to an opiate.

In a particular embodiment, the *P*. *somniferum* seed extract is an extract of the invention.

Generally, such methods allows determining the capacity of an IgE specific to an opiate or to a compound structurally related to an opiate to bind to a *P*. *somniferum* seed extract, specifically to one or more allergenic components (e.g., proteins) of said extract.

"Biological sample susceptible of containing antibodies" is understood as any biological sample susceptible of containing antibodies which can be obtained from a subject; non-limiting illustrative examples of said biological sample include biopsy, tissue, cell or fluid samples, for example, blood, milk, plasma, saliva, serum, etc. In a particular embodiment, said biological sample is selected from blood, plasma or serum, preferably serum.

As used in the present description, "IgE", refers to immunoglobulin E, an antibody involved in allergies, particularly in type I hypersensitivity, such that the recognition of this antibody by mast cells causes their degranulation.

In a particular embodiment, said *in vitro* method for diagnosing whether a subject has an allergy or hypersensitivity to an opiate or to a compound structurally related to an opiate, particularly, to an opiate used in anesthesia, or to NMBA, is carried out by means of an immunoassay.

As used herein, "immunoassay" includes various immunological techniques such as ELISA (Enzyme-Linked Immunosorbent Assay), Western-blot or dot-blot, RIA (radioimmunoassay), immunocytochemical and immunohistochemical techniques, etc.

In a particular embodiment, said immunoassay is an immunoblot or Western blot. This technique is based on the detection of proteins previously separated by gel electrophoresis in denaturing conditions and immobilized in a membrane which is subsequently incubated with one or more antibodies specific for the protein and is detected by means of a system, for example, a chemiluminescent or fluorescent system. Immunofluorescence analysis requires the use of a specific antibody labeled with a fluorescent compound.

In another particular embodiment, said immunoassay is a Dot Blot which starts with the direct application of the sample (through a device in which said deposit is in the shape of a well) in a membrane. The subsequent steps are the same: incubation of the membrane with a serum and the subsequent labeling and developing.

In another additional particular embodiment, the immunoassay is an enzyme immunoassay (EIA), also known as ELISA. This technique is based on the use of antigens or enzyme-labeled antibodies, such that the conjugates formed between the target antigen and the labeled antibody results in the formation of enzymatically active complexes. Since one of the components (the antigen or the labeled antibody) is immobilized on a support, the antibody-antigen complexes are immobilized on the support and can therefore be detected by adding a substrate which is converted by the enzyme into a product that can detected by spectrophotometry or fluorometry, for example.

There are commercially available kits and systems for determining specific IgE [ImmunoCAP®], a fluorometric enzyme immunocapture assay (FEIA) in which the support is a cellulose disc on which the antigens to be assayed are coupled.

In another aspect, the invention relates to a kit, hereinafter kit of the invention, comprising a *P*. *somniferum* seed extract. In a preferred embodiment, said extract is an extract of the invention.

In a particular embodiment, said extract is in the form of a composition comprising a pharmaceutically acceptable vehicle in addition to said extract. In a specific embodiment, said composition is in the form of a solution or suspension in a pharmaceutically acceptable vehicle, for example, saline solution, etc. In another particular embodiment, the extract is in the form of a lyophilisate and the kit provided by this invention contains, if desired, a medium for reconstituting the lyophilisate. In another particular embodiment, said extract is bound to a support. Non-limiting illustrative examples of said supports include columns, filters, plastics, glass slides, silicon, microwells, nitrocellulose or PVDF membranes, magnetic beads, antigen-antibody recognition strips, etc.

In another aspect, the invention relates to the use of a kit of the invention for diagnosing whether a subject has an allergy or hypersensitivity to an opiate or to a compound structurally related to an opiate, particularly an opiate used in anesthesia or a neuromuscular blocking agent.

The invention is illustrated below based on the following examples provided as non-limiting illustrative examples of the scope of the invention.

### Example 1

### Producing water soluble and fat soluble P. somniferum seed extracts

To produce the water soluble and fat soluble *P*. *somniferum* seed extracts, *P. somniferum* seeds were suitably classified and certified as well as standardized in terms of their purity (greater than 99%), and then subjected to extraction processes.

The *P*. *somniferum* seeds were weighed, crushed and suspended in an amount of 0.25 g/ml in a pH 7.4 phosphate buffered saline (PBS) [1.37 mM NaCl, 14.7 mM KH₂PO₄, 78.1 mM Na₂HPO₄, 26.8 mM KCl] and 25% glycerol (v/v). They were then homogenized in cold (5 ± 3°C) by means of using a mortar and pestle and the homogenization of the seeds was maintained until the greatest possible amount of their content was released. The homogenate was kept under magnetic stirring in cold conditions (5 ± 3°C) for 30 minutes. The sample was then sieved to separate the seed debris and the extract was centrifuged in cold conditions (5 ± 3°C) at 10,000 g for 15 minutes, after which the fatty content (fat soluble fraction) was separated from the non-fatty content (water soluble fraction). Said fractions were independently treated for producing the water soluble and fat soluble *P*. *somniferum* extracts.

### Producing the water soluble extract

### Method [1]

To produce the water soluble extract, the water soluble fraction originating from the prior centrifugation was filtered through an AP series AP 20 type glass fiber filter (Millipore®) comprising a glass fiber prefilter and a membrane of 0.8 to 8 µm [AP 2009000 membrane (Millipore®)], and dialyzed in cold conditions (5 ± 3°C) against deionized water with membranes having a molecular weight cut-off of 3,500 Da [Visking (Iberlabo)] for 16 hours. Finally, the dialyzed water soluble extract was lyophilized.

### Method [2]

Alternatively, the water soluble *P*. *somniferum* seed extract can be produced by homogenizing the previously crushed *P*. *somniferum* seeds and defatting them with acetone in a seed:acetone ratio of 2:1 (w/v) for 1 hour in cold conditions (5 ± 3°C). After drying the sample by means of removing the organic fraction made up of acetone and substances soluble in acetone by filtering the mixture, the protein fraction was extracted by suspending the sample (containing the seed debris and substances insoluble in acetone) in 0.1 mol/l of Tris-HCl pH 7.5, 10 mM ethylenediaminetetraacetic acid (EDTA), in a sample:(Tris-HCl + EDTA) ratio of 1:5 (w/v) for 1 hour at 5 ± 3°C. It was then centrifuged at 9,000 g for 30 minutes at 5 ± 3°C. The supernatant was filtered through AP 2009000 membranes (Millipore®) and dialyzed in cold conditions (5 ± 3°C) against deionized water with membranes having a molecular weight cut-off of 3,500 Da [Visking (Iberlabo)] for 16 hours. The dialyzed water soluble extract was lyophilized.

### Producing the fat soluble extract

### Method [A]

To produce the fat soluble extract (fatty content), the fat soluble fraction originating from the first centrifugation was resuspended in cold conditions (5 -+ 3°C) in pH 7.4 PBS and 50% glycerol and then centrifuged at 10,000 g for 5 minutes. This process was performed twice. After washing with buffer and glycerol, an additional washing of the oily phase was performed with pH 9.6 carbonate/bicarbonate buffer. The precipitate containing the fat soluble extract was resuspended in a chloroform:methanol mixture at a ratio 2:1 (v/v) and then centrifuged at 3,000 g for 10 minutes. The organic fraction was collected to which cold acetone (5 ± 3°C) was added at an organic fraction:acetone ratio of 1:2 (v/v) and it was left for 15 minutes at -20°C for the subsequent centrifugation of the sample at 10,000 g for 15 minutes. The precipitated proteins originating from the lipid bodies (fat soluble fraction) are stabilized in cold conditions (-20°C).

### Method [B]

Alternatively, said fatty content can be directly resuspended in cold acetone without passing through chloroform/methanol and subjecting the suspension to the same resting action at -20°C and centrifugation at 10,000 g for 15 minutes. The precipitated proteins originating from the lipid bodies (fat soluble fraction) were stabilized in cold conditions (-20°C).

The water soluble and fat soluble *P*. *somniferum* seed extracts produced previously were standardized to a concentration of 10 and 0.1 mg/ml (expressed in dry lyophilized mass).

Electrophoretic profile of the water soluble and fat soluble *P*.

### somniferum seed extracts

For the purpose of separating the proteins present in the water soluble and fat soluble *P*. *somniferum* seed extracts produced previously, said extracts were subjected to polyacrylamide gel (15%) electrophoresis with sodium dodecyl sulfate (SDS-PAGE) and Coomassie blue stain [Proc Natl Acad Sci USA. 1974; 76:4350-4]. Additionally, the fat soluble *P*. *somniferum* seed extracts produced previously were subjected to two-dimensional electrophoresis, specifically to polyacrylamide gel (15%) electrophoresis with two-dimensional sodium dodecyl sulfate (2D-SDS-PAGE) and Coomassie blue stain. Briefly, the proteins forming part of the fat soluble extract were loaded on gradient strips having pH 3 to 10 (IPG) (ReadyStrips, BioRad, California, USA) for the first dimension (isoelectric focusing) and they were located depending on their isoelectric point (pI) by isoelectric focusing according to the instructions of the apparatus manufacturer. The strips were then first incubated with an equilibration buffer, and then with the same buffer to which iodoacetamide was added. Once equilibrated, the strips were introduced in 15% polyacrylamide gel for their separation in the second dimension (SDS-PAGE). The gel was then stained with Coomassie blue (Coomassie Brilliant Blue R-250). The results obtained are shown in Figures 1 and 2.

Figure 1 shows the electrophoretic profiles of the water soluble *P*. *somniferum* seed extract (i) produced according to method [1] (lane 1), and the fat soluble *P*. *somniferum* seed extract (ii) produced according to method [A] (lane 2), determined by means of 15% SDS-PAGE and Coomassie blue stain. It is worth highlighting the existence of a band of about 52 kDa which may be similar to that identified by Moneo *et al*. [Moneo I. et al., Occupational asthma caused by Papaver somniferum. Allergol Immunopathol 1993; 21(4):145-8] and which may correspond to a major protein, as well as bands of about 20 kDa and 32 kDa [these bands seem to be important specific IgE-binding proteins in the sera of the 6 analyzed patients (Figure 3)] in the water soluble extract. With respect to the fat soluble extract, it is worth highlighting a band with a relative molecular weight of about 16 kDa corresponding with ArgJ protein (involved in the biosynthesis of arginine), band(s) with a relative molecular weight of about 18-20 kDa which may correspond (by relative molecular weight) with the so-called oleosins, a band with a relative molecular weight of about 23 kDa corresponding with an aquaporin, a band with a relative molecular weight of about 28 kDa corresponding with a phytochrome, a band with a relative molecular weight of about 34 kDa corresponding with a caleosin and bands with a relative molecular weight around 40 kDa which would correspond with the isoforms of 11S globulin protein.

Figure 2 shows the electrophoretic profile of the fat soluble *P*. *somniferum* seed extract produced according to method [A], determined by means of 15% one-dimensional SDS-PAGE (1D - Electrophoresis) [Figure 2, right panel, lane 1) and by means of two-dimensional electrophoresis [2D-15% SDS-PAGE] (Figure 2, left panel - 2D Electrophoresis) and Coomassie blue stain in both cases. Figure 4 shows the allergenic profile of the fat soluble *P*. *somniferum* seed extract produced according to method [A], determined by means of 15% two-dimensional SDS-PAGE [2D-15% SDS-PAGE] (Figure 4, left panel - 2D Electrophoresis - Blotting) and Coomassie blue stain and IgE immunoblotting.

### Example 2

### Diagnostic tests for diagnosing allergy to opiates by means of using water soluble and fat soluble P. somniferum seed extracts

### 1. Materials and Methods

### 1.1 Subjects

Immunoglobulin E (IgE)-mediated allergic hypersensitivity in 6 subjects who experienced anaphylaxis during anesthesia was evaluated. The anesthesia administered to said patients generally included atracurium besylate or rocuronium bromide, fentanyl or morphine, thiopental and midazolam, and, for reversion, atropine and naloxone. The patients were recruited based on their medical history and sometimes on a series of diagnostic tests with morphine derivatives.

The participating subjects were selected and evaluated by the Allergy Unit of the Hospital Universitario Rio Hortega (Valladolid, Spain). All the subjects participating in this study gave their informed consents, with the exception of one who died during anesthesia in a surgical intervention and this subject habitually took opiates, analgesics and heroin. The patient died after the administration of atracurium 10 minutes after starting the intervention due to anaphylactic shock. Erythema, hives and hypotension that could not be controlled were observed. This patient did not give his/her consent for tests, in fact the IgE was analyzed in the patient's serum after passing away. The patient had high serum tryptase. The study was approved by the hospital's CEIC [Clinical Trial and Research Committee].

### 1.2 Extracts and controls

The following *P*. *somniferum* seed extracts were used:
a) water soluble extract produced according to method [1] of Example 1 and water soluble extract produced according to method [2] of Example 1;
b) fat soluble extract produced according to method [A] of Example 1; and
c) mixture of water soluble [a)] and fat soluble extracts [b)].

Said extracts were standardized to a concentration of 10 and 0.1 mg/ml (expressed in the amount of protein).

Morphine (morphine chloride) and pholcodine were used as controls.

### 1.3 In vivo diagnostic tests

The water soluble and fat soluble *P*. *somniferum* seed extracts mentioned above were used in:
- skin tests (Prick Test), at a concentration of 5 mg/ml; the concentrations applied were 1 mg/ml, 0.5 mg/ml, 0.1 mg/ml and 0.05 mg/ml (expressed in the amount of protein); and
- bronchial challenge tests in accordance with conventional protocols [Agius R. Opiate inhalation and occupational asthma. BMJ 1988;297(6662):1511-2] at a concentration of 5 mg/ml; the concentrations applied were 1 mg/ml, 0.5 mg/ml, 0.1 mg/ml and 0.05 mg/ml (expressed in the amount of protein).

Solutions of morphine (morphine chloride) and pholcodine at a concentration of 1 mg/ml were used as controls in skin tests (Prick Test).

### 1.4 In vitro diagnostic tests

### 1.4.1 Total serum IgE determination

Serum samples from each patient were subjected to total IgE determination by means of ELISA. To that end, briefly, the ELISA microwells were coated with monoclonal anti-human IgE antibodies and the patients' serum was then added, whereby, in the event that said serum contains IgE, a complex is formed between the immobilized monoclonal anti-human IgE antibody and the IgE possibly present in the patients' serum, which is recognized by an anti-human IgE antibody of an animal (e.g., goat) labeled with an enzyme (e.g., peroxidase); a colored reaction is generated after adding the substrate (e.g., 3,3',5,5'-tetramethylbenzidine (TMB) when the enzyme is peroxidase). The color intensity is directly proportional to the concentration of IgE present in the sample (serum) analyzed.

Alternatively, the total IgE can be quantified using the ImmunoCAP Total IgE kit (Phadia) in which, instead of an allergen, a monoclonal anti-IgE antibody is bound to the system for determining the total IgE. The assay starts by washing the system containing the anti-IgE antibody. The apparatus then dispenses each of the target sera, the standards and the controls. This antigen/antibody reaction phase is incubated for 30 minutes. The anti-IgE antibody covalently bound to the InmunoCAP system reacts with the total IgE of the patient's serum. An anti-IgE antibody labeled with an enzyme (beta-galactosidase) is added after washing and the anti-IgE [test sample] + IgE + enzyme-labeled anti-IgE antibody complex is incubated. Finally, it is washed and a developing solution (4-methyl-umbelliferyl beta-D galactosidase) is added. After stopping the reaction, a reducing substance which releases the enzyme and reacts with a chromogenic substrate to form a colored compound is added. The fluorescence is measured by a fluorometer, which places the values obtained in each test serum on the calibration curve expressing the results in kU/l. The color change of the test sample is directly proportional to the amount of IgE present in the patient's serum. The range of measurement of the system is from 2 to 5,000 kU/l.

### 1.4.2 Specific serum IgE determination

Serum samples from each patient were subjected to specific IgE determination with respect to the water soluble *P*. *somniferum* seed extract produced according to method [1] of Example 1, and with respect to the control solutions of morphine (morphine chloride) and pholcodine, using the ImmunoCAP® specific IgE system (Phadia, Sweden), following the manufacturer's instructions. Briefly, said extract was contacted with the solid phase of the ImmunoCAP® system for the purpose of binding the allergens present in said extracts (e.g., proteins) and the patients' sera were then added, whereby a complex is formed between the immobilized allergen and the specific human IgE present in the patients' sera, which is recognized by an anti-human IgE antibody labeled with an enzyme (beta-galactosidase) contained in the ImmunoCAP® kit used; after incubation, the unbound labeled anti-human IgE antibody is removed and the binding mixture is incubated with a developing agent (4-methyl-umbelliferyl beta-D galactosidase) and after stopping the reaction, the fluorescence of the eluate is measured such that the higher the fluorescence, the more specific IgE there will be in the analyzed sample.

### 1.4.3 SDS-PAGE and IgE immunoblotting

Likewise, serum samples from the patients were subjected to electrophoretic and specific IgE binding techniques with respect to the components of the water soluble and fat soluble *P*. *somniferum* seed extracts separated by molecular size (SDS-PAGE and IgE immunoblotting). The separation of the proteins from said extracts was performed by means of SDS-PAGE as described in Example 1.

Briefly, once the proteins are separated and transferred to a polyvinylidene fluoride (PVDF) membrane, the assay is performed as described in Section 1.4.4, i.e., by adding the sera to be assayed, whereby a complex is formed between the immobilized allergen (e.g., protein) and the specific human IgE present in the patients' sera, which is recognized by an anti-human IgE antibody labeled with an enzyme, specifically, mouse anti-human IgE antibodies labeled with horseradish peroxidase (Mouse Anti-Human IgE (Fc)-HRP (Clone B3102E8) - Southern Biotech), diluted with a blocking buffer at a ratio of 1:1000. After incubation, the unbound labeled anti-human IgE antibody is removed and the binding mixture is incubated with a developing agent, specifically, Western Lightning Plus ECL (Perkin Elmer), a chemiluminescent product containing Luminol and an oxidizing reagent.

### 1.4.4 Dot blot

This technique is based on the detection of proteins previously coupled on a membrane through a device in which said deposit is in the shape of a well which is then incubated with one or more antibodies specific for the protein and detected by means of a system, for example, chemiluminescent or fluorescent system. Chemiluminescent or fluorescent analysis requires the use of a specific antibody labeled with a chemiluminescent or fluorescent compound (Towbin H, Staehelin T, Gordon J. Electrophoretic transfer of proteins from polyacrylamide gels to nitro-cellulose sheets; procedure and some applications. Proc Natl Acad Sci USA. 1974; 76:4350-4).

### Assay 1

The following were analyzed in this assay: (i) sera from 6 patients allergic to morphine derivatives (opiate positive), and (ii) sera originating from patients not allergic to morphine derivatives (opiate negative) [sera from patients with pollen allergy, whose clinical condition corresponded to a symptomatology caused by said pollens; therefore without suspicion of an allergy to opiate derivatives (a pool with 12 sera was prepared)]. Likewise, sera from premature babies, sera from newborns and PBS buffer were used as negative controls. All of the above were at 1:1 dilutions in blocking buffer [0.5% PBS-Tween® 20 (Panreac)].

Briefly, the samples used were proteins originating from the water soluble *P*. *somniferum* seed extract produced according to method [1] of Example 1 and proteins originating from the fat soluble *P*. *somniferum* seed extract produced according to method [A] of Example 1. Said samples were deposited in the well of the device in an amount of 100 ng and 10 ng (amount of protein). The sera to be assayed were then added, whereby a complex is formed between the immobilized allergen (e.g., protein) and the specific human IgE present in the patients' sera, which is recognized by an anti-human IgE antibody labeled with an enzyme, specifically mouse anti-human IgE antibodies labeled with horseradish peroxidase (Mouse Anti-Human IgE (Fc)-HRP (Clone B3102E8) - Southern Biotech) diluted with a blocking buffer at a ratio of 1:1000. After incubation, the unbound labeled anti-human IgE antibody is removed and the binding mixture is incubated with a developing agent, specifically Western Lightning Plus ECL (Perkin Elmer), a chemiluminescent product containing Luminol and an oxidizing reagent. After stopping the reaction the chemiluminescence of the eluate is measured such that the higher the chemiluminescence, the more specific IgE there will be in the analyzed sample.

### Assay 2

Another assay was performed by means of the same technique (dot blot) and using the same samples as in Assay 1, with the exception that in this Assay 2:
- the sample were deposited in the well of the device at a concentration of 1000 ng, 100 ng, 10 ng, and 1 ng of amount of protein per well; and
- the sera to be assayed were: (i) serum from a patient with a serious allergic reaction to the application of atracurium (diluted to 1:4 in blocking buffer (0.5% PBS Tween)); and (ii) a mixture of sera from 4 patients with a serious clinical reaction due to the application of anesthesia: fentanyl, alfentanil, morphine, propofol, droperidol, thiopental and/or ketamine (diluted to 1:4 in blocking buffer (0.5% PBS Tween)). PBS was used as control.

### 2. Results

### 2.1 In vivo diagnostic tests

Five of the patients had positive skin tests and positive bronchial challenge to one or both (water soluble and fat soluble) *P. somniferum* seed extracts. The sixth patient selected died during anesthesia in a surgical intervention and could not be analyzed.

### 2.2 In vitro diagnostic tests

### Total serum IgE determination (ELISA)

The results obtained by means of the ELISA assay for total serum IgE determination clearly showed that the samples from the 6 patients analyzed had high total IgE titers.

### Specific serum IgE determination (ImmunoCAP®)

The results obtained by means of the specific serum IgE determination assay (ImmunoCAP® specific IgE) clearly showed that the serum samples from the 6 tested patients were clearly positive (specific IgE) with respect to the water soluble *P*. *somniferum* seed extract, and the presence of morphine- or pholcodine-specific IgE however was not detected in any of the sera analyzed.

### SDS-PAGE and IgE immunoblotting

Additionally, the serum samples from the 6 patients were assayed by means of immunoblotting techniques with respect to the protein fractions of the water soluble and fat soluble *P*. *somniferum* seed extracts previously separated by their molecular size by means of SDS-PAGE (Example 1). The sera of the six selected subjects recognized several of the proteins from the water soluble and fat soluble extracts (Figures 3 and 4).

### Extract recognition by specific IgE (Dot blot)

The results obtained by means of Assay 1 (Dot Blot) clearly showed that both the water soluble and the fat soluble *P*. *somniferum* seed extract at the assayed concentrations, i.e., the allergenic proteins present in said extracts, are recognized by specific IgE in the group of opiate positive patients [Figure 5].

Likewise, the results obtained by means of Assay 2 (Dot Blot) clearly showed that the fat soluble *P*. *somniferum* seed extract has a higher rate of recognition (with greater intensity) than the water soluble *P*. *somniferum* seed extract at the same amount of protein [Figure 6].

### Example 3

### Diagnostic tests for diagnosing allergy to NMBA by means of using water soluble and fat soluble P. somniferum seed extracts

### 1. Materials and Methods

### 1.1 Subjects

Hypersensitivity to drugs used in anesthesia of a healthy teenager who developed a near-death anaphylactic experience during an appendicitis operation was evaluated. The patient suffered cardiac arrest after the administration of rocuronium NMBA (muscle relaxant). The patient had anti-poppy seed and anti-chicken meat antibodies, even though the patient denied chicken consumption and was fasting as well.

### 1.2 Extracts and controls

The following *P*. *somniferum* seed extracts were used:
a) the water soluble extract produced according to method [1] of Example 1 and the water soluble extract produced according to method [2] of Example 1; and
b) the fat soluble extract produced according to method [A] of Example 1.

Said extracts were standardized to a concentration of 10 and 0.1 mg/ml (expressed in the amount of protein). Morphine (morphine chloride) and pholcodine were use as controls.

### 1.3 In vivo diagnostic tests

The water soluble and fat soluble *P*. *somniferum* seed extracts mentioned above were used in skin tests (Prick Test) at a concentration of 1 mg/ml, 0.5 mg/ml, 0.1 mg/ml and 0.05 mg/ml (expressed in the amount of protein).

Morphine (morphine chloride) and pholcodine solutions at a concentration of 1 mg/ml were used as controls in skin tests (Prick Test).

### 2. Results

The patient had positive skin tests both with respect to water soluble *P*. *somniferum* seed extract (24 mm²) and fat soluble *P*. *somniferum* seed extract (52 mm²).

## Claims

1. Use of *Papaver somniferum* seed extract in the preparation of a reagent for diagnosing allergy or hypersensitivity to an opiate or to a compound structurally related to an opiate.

2. Use according to claim 1, wherein said extract is a water soluble extract, a fat soluble extract or a mixture thereof.

3. Use according to any of claims 1 to 2, wherein said opiate is an opiate used in anesthesia.

4. Use according to any of claims 1 to 2, wherein said opiate is heroin.

5. Use according to any of claims 1 to 2, wherein said compound structurally related to an opiate is a neuromuscular blocking agent (NMBA).

6. A method for producing a water soluble *Papaver somniferum* seed extract comprising the steps of:
a) suspending *Papaver somniferum* seeds in a medium comprising a buffer with a pH comprised between approximately 6 and approximately 9 and glycerol;
b) subjecting the *Papaver somniferum* seeds contained in the suspension obtained in step a) to a homogenization treatment at a temperature comprised between approximately 0°C and approximately 10°C for obtaining a *Papaver somniferum* seed homogenate comprising (i) a solid phase comprising *Papaver somniferum* seed cell debris and (ii) a liquid phase comprising the substances contained in said seeds;
c) subjecting the homogenate obtained in step b) to stirring at a temperature comprised between approximately 0°C and approximately 10°C;
d) separating the solid and liquid phases of the homogenate resulting from step c);
e) centrifuging the liquid phase obtained in step d) between 7,000 and 13,000 g for a time period sufficient to separate a liquid aqueous phase comprising a water soluble *Papaver somniferum* seed extract and a solid or semi-solid oily phase comprising a fat soluble *Papaver somniferum* seed extract formed by the substances contained in the *Papaver somniferum* seeds which are not soluble in an aqueous medium;
f) separating the aqueous phase comprising a water soluble *Papaver somniferum* seed extract obtained in step e);
g) filtering said aqueous phase comprising a water soluble *Papaver somniferum* seed extract separated in step f) through a filter with a pore size comprised between approximately 0.8 micrometers (µm) and approximately 8 µm for obtaining a filtrate comprising a water soluble *Papaver somniferum* seed extract; and
h) subjecting said filtrate comprising a water soluble *Papaver somniferum* seed extract to dialysis against deionized water with membranes having a molecular weight cut-off comprised between approximately 3,500 Da and approximately 7,000 Da at a temperature comprised between approximately 0°C and approximately 10°C for obtaining a water soluble *Papaver somniferum* seed extract.

7. A method for producing a water soluble *Papaver somniferum* seed extract comprising the steps of:
a) contacting *Papaver somniferum* seeds with an organic polar aprotic solvent at a temperature comprised between approximately 0°C and approximately 10°C for obtaining a *Papaver somniferum* seed homogenate comprising a solid phase comprising insoluble *Papaver somniferum* seed debris and a liquid phase comprising said organic polar aprotic solvent and substances originating from said seeds soluble in said organic polar aprotic solvent;
b) separating said liquid phase generated in step a) under conditions which allow leaving the solid phase of step a) substantially free of said liquid phase;
c) contacting said solid phase substantially free of liquid phase of step b) with a medium comprising (i) a buffer with a pH comprised between approximately 6 and approximately 9 and (ii) a chelating agent, at a temperature comprised between approximately 0°C and approximately 10°C for extracting the water soluble fraction from the *Papaver somniferum* seeds and generating a suspension comprising a water soluble *Papaver somniferum* seed extract;
d) centrifuging the suspension obtained in step c) between approximately 6,000 g and approximately 12,000 g for a time period comprised between approximately 15 minutes and approximately 2 hours at a temperature comprised between approximately 0°C and approximately 10°C and separating the supernatant comprising a water soluble *Papaver somniferum* seed extract;
e) filtering the supernatant of step d) comprising a water soluble *Papaver somniferum* seed extract through a filter with a pore size comprised between approximately 0.8 µm and approximately 8 µm for obtaining a filtrate comprising a water soluble *Papaver somniferum* seed extract; and
f) subjecting said filtrate comprising a water soluble *Papaver somniferum* seed extract obtained in step e) to dialysis against deionized water with membranes having a molecular weight cut-off comprised between approximately 3,500 Da and approximately 7,000 Da at a temperature comprised between approximately 0°C and approximately 10°C for producing a water soluble *Papaver somniferum* seed extract.

8. A method for producing a fat soluble *Papaver somniferum* seed extract comprising the steps of:
a) suspending *Papaver somniferum* seeds in a medium comprising a buffer with a pH comprised between approximately 6 and approximately 9 and glycerol;
b) subjecting the *Papaver somniferum* seeds contained in the suspension obtained in step a) to a homogenization treatment at a temperature comprised between approximately 0°C and approximately 10°C for obtaining a *Papaver somniferum* seed homogenate comprising (i) a solid phase comprising *Papaver somniferum* seed cell debris and (ii) a liquid phase comprising the substances contained in said seeds;
c) subjecting the homogenate obtained in step b) to stirring at a temperature comprised between approximately 0°C and approximately 10°C;
d) separating the solid and liquid phases of the homogenate resulting from step c);
e) centrifuging the liquid phase obtained in step d) between 7,000 and 13,000 g for a time period sufficient to separate a liquid aqueous phase comprising a water soluble *Papaver somniferum* seed extract and a solid or semi-solid oily phase comprising a fat soluble *Papaver somniferum* seed extract formed by the substances contained in the *Papaver somniferum* seeds which are not soluble in an aqueous medium;
f) separating the oily phase comprising a fat soluble *Papaver somniferum* seed extract obtained in step e);
g) resuspending the oily phase originating from step f) in a medium comprising (i) a buffer with a pH comprised between approximately 6 and approximately 9 and (ii) glycerol;
h) centrifuging the suspension obtained in step g) between approximately 7,000 g and approximately 13,000 g for a time period comprised between approximately 2 minutes and approximately 15 minutes for obtaining an oily phase comprising a fat soluble *Papaver somniferum* seed extract;
i) resuspending the oily phase originating from step h) in a medium comprising (i) a buffer with a pH comprised between approximately 6 and approximately 9 and (ii) glycerol;
j) centrifuging the suspension obtained in step i) between approximately 7,000 g and approximately 13,000 g for a time period comprised between approximately 2 minutes and approximately 15 minutes for obtaining an oily phase comprising a fat soluble *Papaver somniferum* seed extract;
k) separating the oily phase originating from step j);
l) resuspending the oily phase originating from step k) with a medium comprising at least one organic solvent;
m) centrifuging the suspension obtained in step l) between approximately 2,000 g and approximately 5,000 g for a time period comprised between approximately 5 minutes and approximately 15 minutes for obtaining an oily phase comprising a fat soluble *Papaver somniferum* seed extract;
n) separating the oily phase originating from step m);
o) adding an organic solvent to the oily phase originating from step n) at a temperature comprised between approximately -20°C and approximately 10°C and maintaining at a temperature comprised between approximately -20°C and approximately 10°C for a time period comprised between approximately 5 minutes and approximately 25 minutes; and
p) centrifuging the suspension resulting from step o) between approximately 7,000 g and approximately 10,000 g, preferably at about 10,000 g, for a time period comprised between approximately 5 minutes and approximately 30 minutes for producing a fat soluble *Papaver somniferum* seed extract.

9. A method for producing a fat soluble *Papaver somniferum* seed extract comprising the steps of:
a) resuspending the oily phase originating from step f) of the production method [A] for producing a fat soluble extract of the invention in cold acetone at a temperature comprised between approximately -20°C and approximately 10°C and maintaining at a temperature comprised between approximately -20°C and approximately 10°C for a time period comprised between approximately 5 minutes and approximately 25 minutes; and
b) centrifuging the suspension resulting from step o) between approximately 7,000 g and approximately 10,000 g, preferably at about 10,000 g, for a time period comprised between approximately 5 minutes and approximately 30 minutes for producing a fat soluble *Papaver somniferum* seed extract.

10. A *Papaver somniferum* seed extract obtainable according to the method defined in any of claims 6 to 9.

11. An *in vitro* method for diagnosing whether a subject has an allergy or hypersensitivity to an opiate or to a compound structurally related to an opiate which comprises:
a) contacting a biological sample from said subject susceptible of containing antibodies with an extract according to any of claims 1 and 10; and
b) analyzing whether an interaction occurred between an IgE specific to said opiate or compound structurally related to an opiate possibly present in said biological sample susceptible of containing antibodies and said extract;
wherein an interaction between said IgE specific to said opiate and said extract is indicative that said subject has an allergy or hypersensitivity to said opiate.

12. The method according to claim 11, wherein said biological sample susceptible of containing antibodies is a blood sample or a serum sample.

13. A kit comprising an extract according to any of claims 1 and 10.

14. Use of a kit according to claim 13 for diagnosing whether a subject has an allergy or hypersensitivity to an opiate or to a compound structurally related to an opiate.

15. Use of a kit according to claim 14 for diagnosing whether a subject has an allergy or hypersensitivity to an opiate used in anesthesia or to a neuromuscular blocking agent (NMBA).
